## Europäisches Patentamt
## European Patent Office
## Office européen des brevets

(19)

(11) Numéro de publication: **0 181 851**
**A1**

(12)
# DEMANDE DE BREVET EUROPEEN

(21) Numéro de dépôt: 85870138.6

(22) Date de dépôt: 10.10.85

(51) Int. Cl.⁴: **C 12 P 21/00**
**C 12 N 15/00, G 01 N 33/577**

(30) Priorité: 10.10.84 LU 85581

(43) Date de publication de la demande:
21.05.86 Bulletin 86/21

(84) Etats contractants désignés:
AT BE CH DE FR GB IT LI NL SE

(71) Demandeur: S.A. LABORATOIRES S.M.B.
Rue de la Pastorale, 26-28
B-1080 Bruxelles(BE)

(72) Inventeur: Schnek, Arthur G.
Avenue A. Huysmans 75
B-1050 Bruxelles(BE)

(72) Inventeur: de Vries, Ghislaine M.
Avenue des Muguets 26
B-1420 Braine-L'Alleud(BE)

(72) Inventeur: Urbain, Jacques
Tienne du Peuthy 1
B-1338 Lasne(BE)

(72) Inventeur: Bruyns, épousse Etienne Catherine
Rue de la Pêcherie, 178
B-1180 Bruxelles(BE)

(72) Inventeur: Van Acker, Anne-Marie
Avenue d'Orbaix 21
B-1180 Bruxelles(BE)

(72) Inventeur: Fossion, Jacques
Rue cours d'Eau 18
B-1428 Lillois(BE)

(72) Inventeur: Baudier, Philippe
Avenue Blücher 10
B-1410 Waterloo(BE)

(74) Mandataire: De Brabanter, Maurice et al,
Bureau VANDER HAEGHEN 63 Avenue de la Toison d'Or
B-1060 Bruxelles(BE)

(54) Ensemble d'anticorps monoclonaux diriges contre la lactoferrine humaine et le lysozyme humain.

(57) Ensembles d'anticorps monoclonaux dirigés respectivement contre la lactoferrine de lait humain purifiée et contre le lysozyme humain purifié.

Ces ensembles contiennent au moins deux anticorps monoclonaux dirigés soit contre la lactoferrine humaine, soit contre le lysozyme humain.

EP 0 181 851 A1

Croydon Printing Company Ltd.

## Ensembles d'anticorps monoclonaux dirigés contre la lactoferrine humaine et le lysozyme humain

La présente invention concerne des ensembles d'anticorps monoclonaux dirigés respectivement contre la lactoferrine humaine et contre le lysozyme humain.

Lactoferrine et lysozyme :

La lactoferrine et le lysozyme se retrouvent dans de nombreuses sécrétions telles que le lait, la salive, les larmes, les fluides pancréatiques, l'urine, la bile et les fluides synoviaux. Leur présence a été détectée dans de nombreux tissus parmi lesquels nous citerons les poumons, les cellules gastriques et duodénales, le tractus génital et les yeux. (S.A.C. Sykes, M.J. Thomas, D.J. Goldie et G.M. Turner, Clin. Chim. Acta 122 (1982) 385-393, T.L. Peeters, Y.R. Depraetere et G.R. Vantrappen, Clin. Chem. 24 (1978) 2155-2157, R.M. Bennett et J.L. Skosey, Arthritis and Rheumatism 20 (1977) 84-90). L'association des deux antigènes a été décrite notamment dans les granules de leucocytes polymorphonucléaires (M.S. Lefell et J.K. Spitznagel Infect. Immun. 6 (1972) 1761).

Lactoferrine :

La lactoferrine est une glycoprotéine de 70.000 daltons (poids moléculaire). Elle est synthétisée dans les leucocytes polymorphonucléaires neutrophiles et le lait. Elle exerce des propriétés bactériostatiques (Bullen C.L. et Willie A.T., Br. Med. J. 3 (1971) 338). Le taux de lactoferrine est modifié au cours d'infections (J. Breton-Gorius, D.Y. Masson, D. Buriot, J.L. Vilde et C. Griscelli, J. Pedia-trics 94 (1979) 1 - 8), au cours de maladies pancréatiques (S.S. Fedail, P.R. Salmon, R.F. Harvey, A.E. Read, The Lancet 28 (1978) 181-182). Le taux de lactoferrine est également perturbé dans certaines maladies inflammatoires et il reflète "l'activité inflammatoire" (R.M. Bennett et S.L. Skosey, Arthritis and Rheumatism 20 (1977) 84-90).

Lysozyme :

Le lysozyme est une protéine de faible poids moléculaire (14.000 daltons). Il est produit en quantités relativement importantes dans les cellules phagocytaires, les leucocytes polymorphonucléaires, les macrophages. C'est un catalyseur puissant de la destruction de certaines parois bactériennes et il pourrait intervenir dans l'immunité non spécifique. La production de lysozyme varie dans la leucémie et la sarcoïdose, dans des affections gastro-intestinales, rénales et urinaires et dans le rejet de greffes (M.J. Thomas, A. Russo, P. Craswell, M. Ward et I. Steinhardt, Clin. Chem. 27 (1981) 1223-1226).

Dans certains cas, le dosage indépendant des deux protéines, la lactoferrine et le lysozyme, présente un intérêt du fait que les deux protéines n'ont pas exactement la même localisation et que la mesure de leur rapport peut aider à établir un diagnostic.

Bien que l'on puisse obtenir facilement des anticorps polyclonaux contre l'une ou l'autre protéine, les méthodes de dosage utilisant de tels anticorps manquent de précision. Dès lors, l'obtention d'anticorps monoclonaux spécifiques du lysozyme et de la lactoferrine permettrait la mise au point de systèmes de dosage plus sensibles de ces antigènes et conduirait à une amélioration de la détection des nombreuses affections mentionnées précédemment.

L'invention concerne des ensembles d'anticorps monoclonaux dirigés contre des protéines de lait humain purifiées, respectivement contre la lactoferrine et le lysozyme.

Préparation :

La préparation des anticorps monoclonaux s'effectue selon une technique de fusion cellulaire décrite par Köhler G. et Milstein C. (Eur. J. Immunol. 6 (1975), 511 et Nature 256 (1975) 495-497 et modifiée par Franssen J.D., Hérion P. et Urbain J. (Protides of Biol. Fluids 29 (1981) 645, Ed. Peeters, Pergamon Press).

La présente invention couvre également l'application, pour la production des ensembles d'anticorps monoclonaux précités, du procédé caractérisé par l'immunisation de souris au moyen de l'antigène - soit la lactoferrine de lait humain, soit le lysozyme de lait humain, par la fusion cellulaire des cellules de rate de souris immunisée soit avec la lactoferrine, soit avec le lysozyme avec des cellules de myélome de souris sélectionnées, telles que des plasmacytomes de souris BALB/c non sécréteurs, par la sélection des hybridomes sécréteurs d'anticorps spécifiques, soit de la lactoferrine, soit du lysozyme, par le clonage de ces hybridomes, par la production in vivo des anticorps monoclonaux des deux spécificités en ascite, par l'identification des anticorps sécrétés par ces clones et par la caractérisation des classes d'immunoglobulines de ces anticorps, par l'étude de l'affinité relative des anticorps monoclonaux et de leur spécificité épitopique.

L'application du procédé susdit est décrite, à titre non limitatif, dans l'exemple suivant :

1. **Immunisation des souris**

Les antigènes ont été purifiés à partir de lait humain provenant de donneuses saines et leur homogénéité a été testée par électrophorèse sur gel d'acrylamide.

Les préparations d'antigènes purifiés ont été utilisées pour immuniser des souris femelles (souches BALB/c) âgées de 8 semaines.

On immunise d'abord les souris avec 200 µg d'antigène émulsifié dans l'adjuvant complet de Freund et on injecte le mélange par moitié par voie sous-cutanée en points multiples dans le dos et par moitié par voie intra-péritonéale. Deux semaines plus tard, les souris reçoivent une injection de rappel identique. Après une période de repos de 1 mois, les souris reçoivent au jour moins quatre avant la fusion, une injection de 20 µg d'antigène (dans du NaCl 0,15 M) par voie intraveineuse.

Au jour zéro, on sacrifie les souris et on prélève la rate pour la préparation des cellules destinées aux fusions cellulaires.

2. **Cellules de myélome de souris**

La méthode décrite par Franssen J.D., Hérion P. et Urbain J. (Protides of Biol. Fluids 29 (1981) 645, Ed. Peeters, Pergamon Press) a été utilisée.

On a sélectionné des cellules de myélome de souris possédant les propriétés suivantes : croissance rapide in vivo et in vitro, capacité de pouvoir être clonées sans le support de cellules nourricières et en milieu semi-solide, capacité de fusionner à taux élevé. Cette lignée a été développée par Shulman M., Wilde C.D. et Köhler G. (1978) Nature 276, 269-270.

Dans ce but, des cellules myélomateuses de souris, telles que des plasmacytomes de souris BALB/c non sécréteurs, ont été clonées en agar. Les cellules capables de pousser dans ces conditions ont alors été reclonées en agar puis injectées par voie intrapéritonéale dans des souris syngéniques.

Après une à deux semaines, les cellules myélomateuses se développent sous forme de tumeur dans les souris injectées. Les cellules sont alors collectées dans la cavité péritonéale, resélectionnées, reclonées en agar et essayées dans une expérience de fusion cellulaire. Le cycle décrit ci-dessus est recommencé trois fois. A l'issue du procédé, on obtient des sous-clones sélectionnés de la lignée myélomateuse, SP 2/0 - Ag 14, possédant les propriétés voulues pour leur fusion cellulaire ultérieure.

3. Fusion cellulaire Pour effectuer les fusions, on a utilisé les matières et milieux suivants. On a obtenu chez GIBCO le milieu DMEM que l'on a complété avec du sérum de cheval (10 %), du sérum de veau (5 %), des acides aminés non essentiels, du pyruvate de sodium (1 mM), de la pénicilline (100 U/ml) et de la streptomycine (100 µg/ml).

Le milieu de sélection complet (HAT) se composait du milieu décrit ci-dessus ainsi que d'hypoxanthine (10-4 M), d'aminoptérine ($4.10^{-7}$) et de thymidine ($1.5.10^{-5}$ M).

Le milieu solide contenant l'agar (1,5 %) comportait les ingrédients décrits ci-dessus. Les cellules ont été cultivées à 37°C dans une atmosphère humide de 5 % $CO_2$ et 95 % d'air.

La fusion avec des cellules provenant d'une lignée cellulaire de myélome de souris (sous-clones sélectionnés SP 2/0 - Ag 14) a été réalisée selon le procédé de Franssen J.D., Hérion P. et Urbain J. (1981), Proc. XXIX Colloquium on Protides of the Biological Fluids, Peeters Ed.

Selon ce protocole, $3.5\ 10^8$ cellules de rate de souris ont été fusionnées avec $3.10^6$ cellules de myélome de souris de la lignée SP 2/0 - Ag 14. Pour chaque fusion, on distribue les cellules fusionnées dans des plaques de microculture à 96 puits en effectuant des dilutions. Les cultures sont régulièrement alimentées par du milieu frais. Au jour 15 après la fusion, 50 µl de surnageant de chaque culture est testé dans un immuno essai en phase solide pour détecter la sécrétion d'anticorps.

4. **Sélection d'hybridomes sécréteurs d'anticorps spécifiques**

Les anticorps spécifiques de la lactoferrine et les anticorps spécifiques du lysozyme sont détectés par le test ELISA "Enzyme-linked immunosorbent assay" suivant : 50 µl d'une solution d'antigène (2 µg/ml de lactoferrine humaine purifiée ou 2 µg/ml de lysozyme humain purifié) dans du tampon PBS (0,85 % de NaCl, 0,15 M phosphate) pH 7,4 sont incubés dans des puits de plaques en PVC à 96 puits durant une nuit à 4°C. Les sites d'absorption restant sont alors saturés par incubation avec 50 µl de tampon PBS pH 7,4 contenant 1 % d'albumine bovine. Les plaques sont alors lavées plusieurs fois avec du tampon PBS pH 7,4 contenant 0,1 % d'albumine bovine. Les surnageants de culture d'hybridomes ou les dilutions d'antisérum de souris sont mis à réagir avec l'antigène adsorbé dans les cupules durant une nuit à 4°C.

Les anticorps fixés sont alors révélés par des immunoglobulines de chèvre anti-immunoglobulines de souris marquées à la peroxydase (O'Sullivan M.J. et Marks V., Methods in Enzymology 73 (1981) 147) en solution à 1 µg par ml dans du tampon PBS pH 7,5 contenant 10 % de sérum de cheval, 1 % d'albumine bovine et 0,1 % de Tween 80 (Polyéthylène Sorbitan monooléate, USB, Cleveland, Ohio, U.S.A.). L'incubation des réactifs précités se poursuit pendant 4 heures.

Après plusieurs lavages par du tampon PBS pH 7,5, la peroxydase conjuguée est mise en évidence par addition de 50 µl du substrat chromogène (0,4 mg/ml d'ortho-phénylènediamine contenant 0,2 mg/ml de peroxyde d'urée dans du tampon citrate 0,1 M pH 5).

La réaction est arrêtée par addition de 50 µl d'HCl 5N et l'absorbance est lue à 492 nm dans un lecteur automatique (Dynatech AM 120).

5. Clonage et identification Les cellules d'hybridomes donnant une réaction positive soit dans l'enzymoimmunoessai caractéristique de la lactoferrine, soit dans l'enzymoimmunoessai caractéristique du lysozyme sont propagées puis clonées en agarose selon Franssen J.D. et al (1981) (cf. référence point 2). Les clones qui sécrètent des immunoglobulines in situ dans l'agarose sont retestés par l'enzymoimmunoessai décrit au point 4 pour la sécrétion d'anticorps spécifiques respectivement de la lactoferrine et du lysozyme et soumis à un nouveau clonage afin d'assurer la monoclonalité. Ce protocole permet de récupérer au moins deux hybridomes distincts sécrétant des anticorps dirigés contre la lactoferrine humaine et au moins deux hybridomes distincts sécrétant des anticorps dirigés contre le lysozyme.

6. Production d'anticorps monoclonaux en ascite

$2.10^6$ cellules d'hybridomes clonées sont injectées intrapéritonéalement dans des souris (syngéniques ou F1 progeny) qui ont été traitées au pristane (2, 6, 10, 14 tétraméthyl-pentadécane).

7. <u>Purification des anticorps monoclonaux</u>
Le procédé décrit par Stachelin T., Mobbs D.S.,
Hsiang-Fu K., Chu-yen L. et Petska S. (1981),
J. Biol. Chem. 256, 9750-9754, a été suivi dans sa
totalité, si ce n'est que la diéthylaminoéthyl (DEAE)-
cellulose a été remplacée par la DEAE - Affi-gel Blue
(Biorad) pour éliminer les protéases. Les opérations
se font à 4°C. Les fractions d'anticorps monoclonaux
sont recueillies, précipitées deux fois au sulfate de
sodium solide jusqu'à obtenir une concentration finale
de 18 % de ce sel et dialysées contre un tampon carbonate 0,1 M pH 8,5.

8. <u>Caractéristiques des anticorps monoclonaux</u>
La classe d'immunoglobuline à laquelle appartiennent
les anticorps monoclonaux est déterminée par test
d'Ouchterlony.

0181851

REVENDICATIONS

1. Ensembles d'anticorps monoclonaux dirigés respectivement contre la lactoferrine de lait humain purifiée et contre le lysozyme humain purifié.

2. Ensemble d'anticorps monoclonaux selon la revendication 1, caractérisé en ce qu'il contient au moins deux anticorps monoclonaux dirigés contre la lactoferrine humaine.

3. Ensemble d'anticorps monoclonaux selon la revendication 1, caractérisé en ce qu'il contient au moins deux anticorps monoclonaux dirigés contre le lysozyme humain.

4. Application, pour la production d'ensembles d'anticorps monoclonaux dirigés respectivement contre la lactoferrine de lait humain purifiée et contre le lysozyme de lait humain purifié selon l'une quelconque des revendications 1 à 3, du procédé caractérisé par l'immunisation de souris au moyen de l'antigène - soit la lactoferrine de lait humain, soit le lysozyme de lait humain, par la fusion cellulaire des cellules de rate de souris immunisée soit avec la lactoferrine, soit avec le lysozyme avec des cellules de myélome de souris sélectionnées, telles que des plasmacytomes de souris BALB/c non sécréteurs, par la sélection de hybridomes sécréteurs d'anticorps spécifiques, soit de la lactoferrine, soit du lysozyme, par le clonage de ces hybridomes, par la production in vivo des anticorps monoclonaux des deux spécificités en ascite, par l'identification des anticorps sécrétés par ces clones et par la caractérisation des classes d'immunoglobulines de ces anticorps, par l'étude de l'affinité relative des anticorps monoclonaux et de leur spécificité épitopique.

5. Utilisation des ensembles d'anticorps monoclonaux suivant l'une quelconque des revendications 1 à 3, pour la détection d'infections bactériennes, de maladies inflammatoires, de tumeurs malignes, de maladies du sang, d'affections gastrointestinales, rénales et urinaires.

6. Utilisation des ensembles d'anticorps monoclonaux suivant l'une quelconque des revendications 1 à 3, pour la purification des antigènes - la lactoferrine et le lysozyme.

Office européen
des brevets

**RAPPORT DE RECHERCHE EUROPEENNE**

0181851
Numero de la demande

EP 85 87 0138

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | BIOLOGICAL ABSTRACTS, vol. 78, no. 12, décembre 1984, abrégé 91069, Philadelphia, US; S.J. SMITH-GILL et al.: "Antigenic regions defined by monoclonal antibodies correspond to structural domains of avian lysozyme" & J. IMMUNOL. 133(1): 384-393, 1984 * En entier * | 1-4 | C 12 P 21/00 C 12 N 15/00 G 01 N 33/577 |
| | --- | | |
| A | CHEMICAL ABSTRACTS, vol. 100, no. 15, avril 1984, page 284, abrégé 117484k, Columbus, Ohio, US; & JP - A - 58 201 067 (AMANO PHARMACEUTICAL CO., LTD.) 22.11.1983 * En entier * | 1-4 | |
| | --- | | **DOMAINES TECHNIQUES RECHERCHES (Int. Cl.4)** |
| A | BE-A- 896 253 (REGION WALLONE) * Pages 4-6 * | 4 | |
| | --- | | G 01 N |
| A | CHEMICAL ABSTRACTS, vol.95, no. 9, 31 août 1981, page 343, abrégé 75925a, Columbus, Ohio, US; M.J. THOMAS et al.: "Radioimmunoassay for serum and urinary lysozyme" & CLIN. CHEM. (WINSTON-SALEM, N.C.) 1981, 27(7), 1223-6 * En entier * | 1,3,5 | C 12 P A 61 K C 12 Q |
| | --- -/- | | |

Le présent rapport de recherche a été établi pour toutes les revendications

| Lieu de la recherche | Date d'achèvement de la recherche | Examinateur |
|---|---|---|
| LA HAYE | 22-01-1986 | OSBORNE H.H. |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | CHEMICAL ABSTRACTS, vol. 88, no. 21, 22 mai 1978, page 264, abrégé 148485z, Columbus, Ohio, US; S.S. FEDAIL et al.: "Radioimmunoassay of lactoferrin in pancreatic juice as a test for pancreatic diseases" & LANCET 1978, 1(8057), 181-2 * En entier * | 1,2,5 | |

-----

DOMAINES TECHNIQUES
RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 22-01-1986 | OSBORNE H.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82